# EUROPEAN PATENT SPECIFICATION

(11) **EP 2 435 464 B1**
(45) Date of publication and mention of the grant of the patent: **16.07.2014**
(21) Application number: 10724365.1
(22) Date of filing: 27.05.2010
(51) Int. Cl.: C07K 1/107, C07D 249/18, C07D 471/04

(54) **Cross-linking reagents for molecular interaction analysis**
Vernetzungsreagenzien für die molekulare Wechselwirkungsanalyse
Réactifs de réticulation pour l'analyse d'interactions moléculaires

(30) Priority: 29.05.2009 EP 09161580
(43) Date of publication of application: 04.04.2012
(73) Proprietor: Covalx AG, 8952 Schlieren (CH); ETH Zurich, 8092 Zürich (CH)
(72) Inventor: BICH, Claudia, 84140 Alfortville (FR); NAZABAL, Alexis, CH-8003 Zürich (CH); WENZEL, Ryan, Melrose, Massachusetts 02176 (US); ZENOBI, Renato, CH-8049 Zürich (CH)
(74) Representative: Becker, Konrad
(86) International application number: PCT/EP2010/057350
(87) International publication number: WO 2010/136539

(56) References cited:
- WO-A-94/07910
- WO-A-03/099858
- GRY H DIHAZI AND ANDREA SINZ: "Mapping low-resolution three-dimensional protein structures using chemical cross-linking and Fourier transform ion-cyclotron resonance mass spectrometry" RAPID COMMUNICATIONS IN MASS SPECTROMETRY, vol. 17, 15 September 2003 (2003-09-15), pages 2005-2014, XP002535028 cited in the application

## Description

### Field of the invention

The present invention relates to the use of specially designed cross-linking reagents and cross-linking mixtures to improve efficiency and kinetics properties of cross-linking reactions for molecular interactions analysis.

### Background of the invention

Multi-protein complexes are responsible for most important cellular processes. The study of protein complexes is a key element for the elucidation of the cellular machinery. Chemical cross-linking has been largely used for more than twenty years for analyzing protein interactions (Mattson et al., 1993, Mol. Biol. Rep. 17:167-183) allowing the stabilization of these fragile supramolecular structures. However, several drawbacks are associated with the use of these chemical reagents. For instance, the efficiency of the cross-linking reaction is dependent on the quaternary structure of the protein complex targeted, e.g. the spatial position of the amino acids participating in the cross-linking reaction. This efficiency is also dependent on a fast reaction of the cross-linking reagents, minimizing the risk of disruption of the non-covalent interaction during the incubation time. Substantial modification of nucleophilic side chains, such as the acylation of lysines, on the surface of the proteins during the extended incubation times required by typical cross-linking reagents of the state of the art raises the concern of artifactual results due to structural destabilization. The use of state of the art cross-linkers is also subject of many other issues including slow kinetics, low efficiency, lack of stability, and lack of specificity. Additionally, a lot of cross-linkers are only soluble in organic solvents, making the reactions difficult when mixed with aqueous samples containing compounds with weak protein interactions.

Chemical cross-linking has been used for many years in combination with different analytical technologies. Gel electrophoresis has been largely used in combination with cross-linking chemistry and allows the characterization of non-covalent interactions with a relatively simple protocol. In recent work, gel electrophoresis has been used to analyze *in vivo* cross-linked protein interactions (Nowak et al., 2005, Biotechniques 39:715-725; Suchenek et al., 2005, Nat. Methods 2(4):261-267). Although gel electrophoresis is a standard method to analyze cross-linked products, the low resolution of the technique and the difficulty to characterize high molecular weight protein interactions limits its use for most of the biologically relevant protein complexes.

Mass spectrometry has also been used to analyze cross-linked products from protein complex samples. Most of the studies using mass spectrometry are focusing on the topological analysis of the interacting regions. The method is using enzymatic proteolysis and high resolution mass spectrometric analysis of the cross-linked peptides generated (Sinz, A., 2003, J. Mass Spectrom. 38:1225-1237; Dihazi, G.H. et al., 2003, Rapid Commun. Mass Spectrom. 2005-2014). Mass spectrometry has also been used to characterize intact protein complexes stabilized by cross-linking. The use of mass spectrometry allows faster and more accurate characterization of the intact cross-linked protein complexes, taking benefits from the high resolution of this technology compared with gel based techniques. The method was originally developed by Caprioli et al. in the beginning of the 90s and allowed the direct analysis of homo-multimers up to 250 kDa (Farmer, T.B. et al., 1991, Biol Mass Spectrom. 20:796). Although promising, the method was limited to a few exceptional protein complexes, requiring high concentration and relatively small molecular weight (lower than 250 kDa). Recently, a major improvement was introduced by using both High-Mass MALDI technology and an optimized mixture of cross-linking reagents (WO2006/116893; Nazabal et al., 2006, Anal. Chem. 78(11):3562-3570). High-Mass MALDI technology allows high sensitivity measurement of homo; hetero or complicated cross-linked protein complexes with high sensitivity up to 1200 kDa.

A key element of the method using High-Mass MALDI mass spectrometry for the characterization of intact protein complexes is the mixture of cross-linking reagents used. In order to increase the efficiency of the cross-linking reaction, reagents having different spacer length were used (WO2006/116893). The use of multi-length mixture increases the probability to covalently bind two interacting protein and subsequently the sensitivity of detection for the intact complex.

There is a further need for the development of cross-linking reagents that improve the efficiency and kinetics of the cross-linking reaction leading to better detection of intact protein complexes and protein interactions.

### Summary of the Invention

The invention relates to a method of cross-linking proteins and/or other compounds bearing amino functions, wherein the proteins or other compounds or mixtures thereof are incubated with a cross-linker selected from 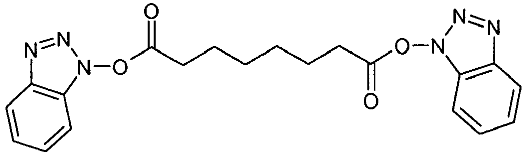 *1*,*1'*-(suberoyldioxy)bisbenzotriazole SBBT (di(*1H*-benzo[*d*][1,2,3]triazol-1-yl) octanedioate), 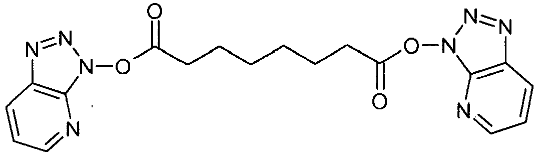 *1,1'*-(suberoyldioxy)bisazabenzotriazole SBAT (di(3H-[1,2,3]triazolo[4,5-b]pyridin-3-yl) octanedioate),
and a mixture of such cross-linkers with each other or with further cross-linkers.

Furthermore, the invention relates to the novel compound *1,1'*-(suberoyldioxy)-bisbenzotriazole (SBBT).

### Brief Description of Figures

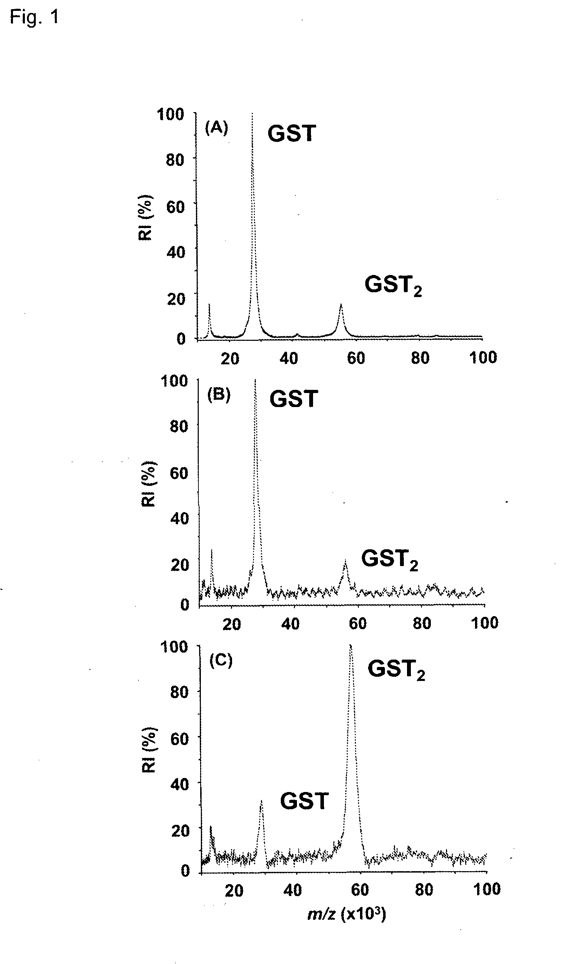

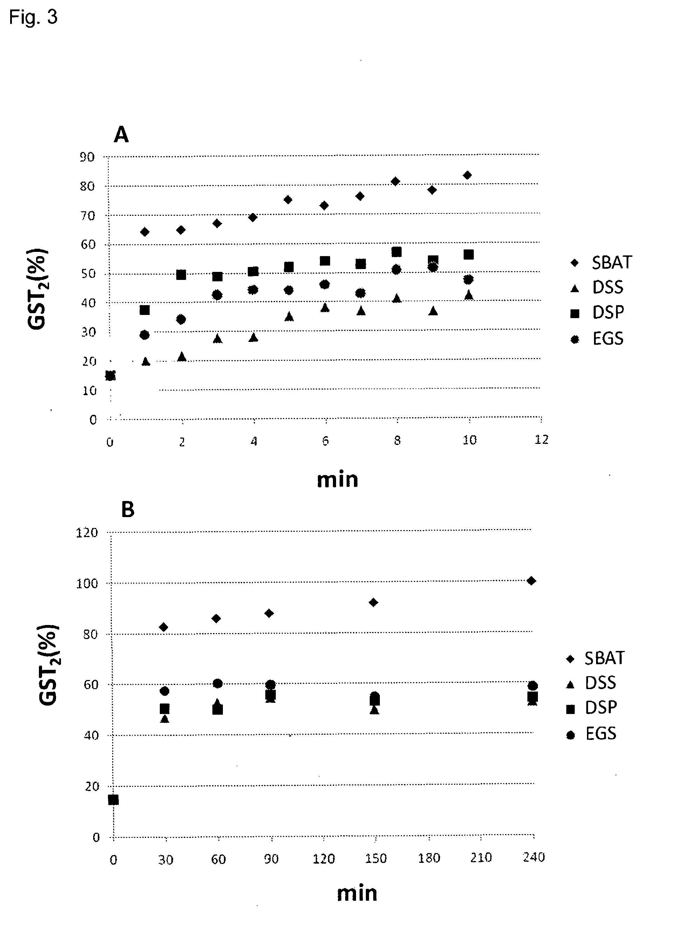

*Figure 1**: High-Mass MALDI mass spectra of glutathione S-transferase (GST).*
   A. Control experiment without cross-linking.
   B. High-Mass MALDI spectrum after 2 min incubation time with disuccinimidyl suberate (DSS) cross-linker.
   C. High-Mass MALDI spectrum after 2 min incubation time with SBAT cross-linker
      (Example 5)
      RI = relative intensity
*Figure 2**: Test of specificity of cross-linker SBAT.*
   High-Mass MALDI analysis of a mixture of glutathione S-transferase (GST) and ubiquitin (U) after 120 min incubation time with cross-linker SBAT (Example 6).
   RI = relative intensity, GST₂ = GST dimer
*Figure 3**: Comparative kinetic study of single cross-linkers*
   Cross-linkers 1,1'-(suberoyldioxy) bisazabenzotriazole (SBAT, ◆), disuccinimidyl suberate (DSS, ▲), dithiobis[succinimidyl propionate] (DSP, ■) and ethylene glycol bis[succinimidylsuccinate] (EGS, ●) are compared for the interaction analysis of glutathione S-transferase (Example 3). The percentage of GST dimer (GST₂) detected is determined directly from the High-Mass MALDI spectra after incubation of the different cross-linkers for 0-10 min (Figure 3A) or 0-240 min (Figure 3B).

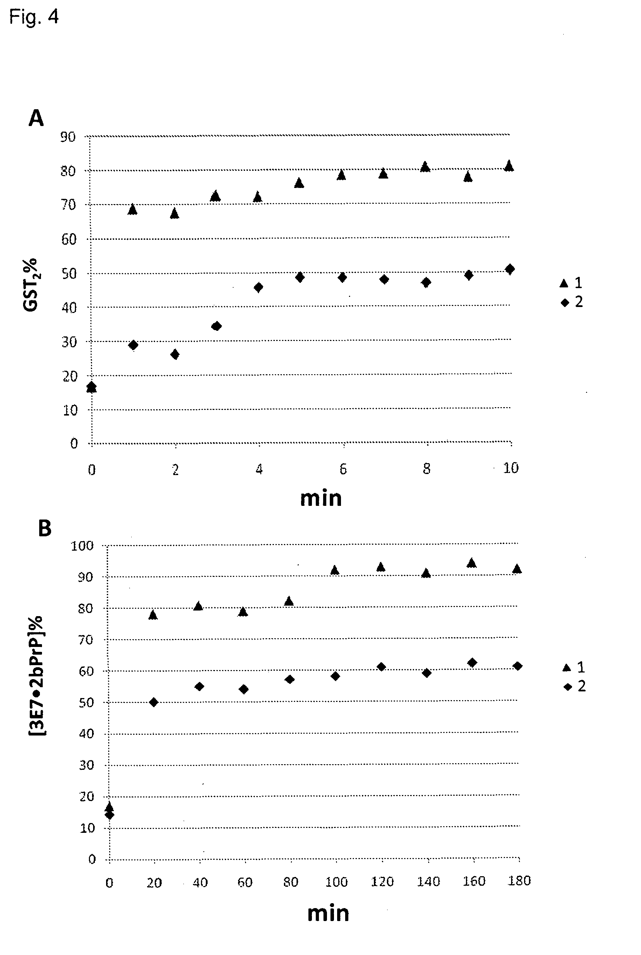

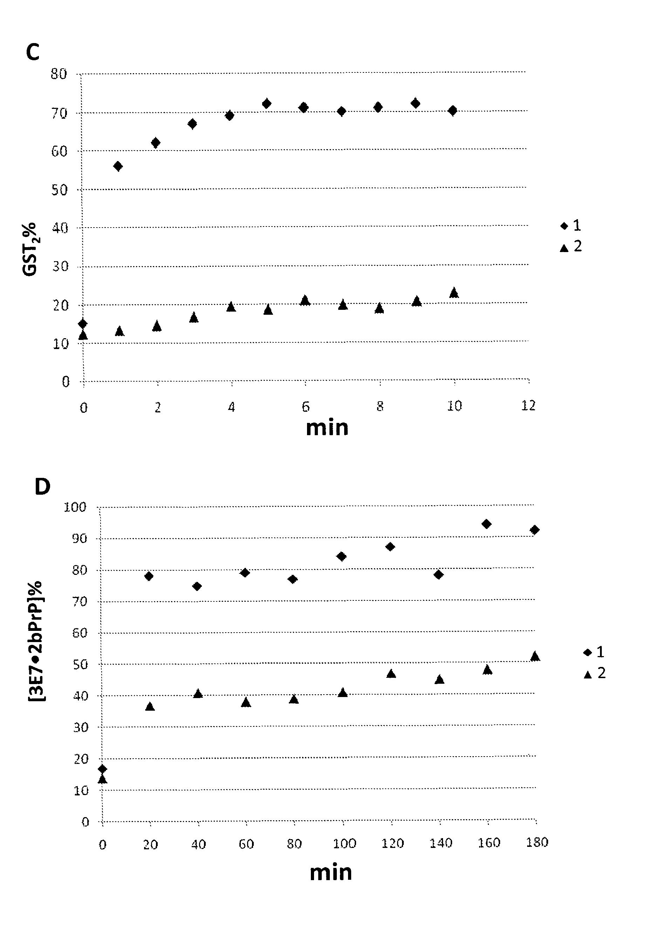

*Figure 4**: Comparative kinetic study of a mixture of cross-linkers having 1-hydroxy-7-azabenzotriazole groups* as *reactive groups*
   Mix 1 (1, ▲) is a mixture of glutaroyldioxy bisazabenzotriazole (GBAT), 1,1'-suberoyldioxy bisazabenzotriazole (SBAT) and decanoyldioxy bisazabenzotriazole (DBAT). Mix 2 (2, ◆) is a mixture of disuccinimidyl suberate (DSS), dithiobis[succinimidyl propionate] (DSP) and ethylene glycol bis[succinimidylsuccinate] (EGS). Each mixture is tested for the interaction analysis of glutathione S-transferase (Figure 4 A,B) and for the immuno-complex [6H4•2bPrP] (Figure 4 C,D). The percentage of GST dimer (GST₂) or [6H4•2bPrP] immuno-complex detected is determined directly from the High-Mass MALDI spectra after incubation for 0-10 min (Figure 4 A,C) or 0-240 min (Figure 4 B,D) (Example 8).

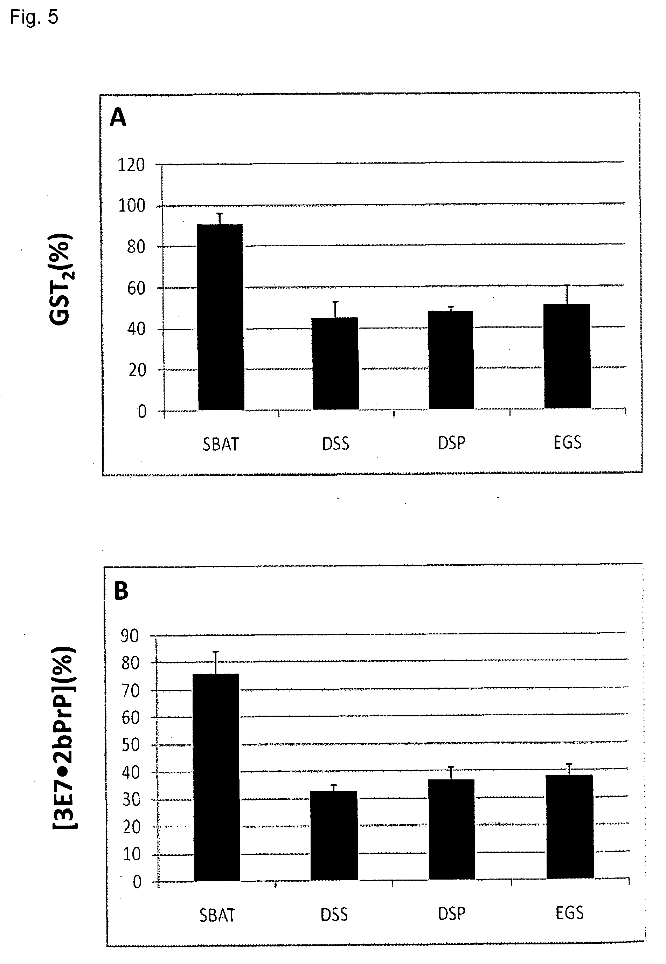

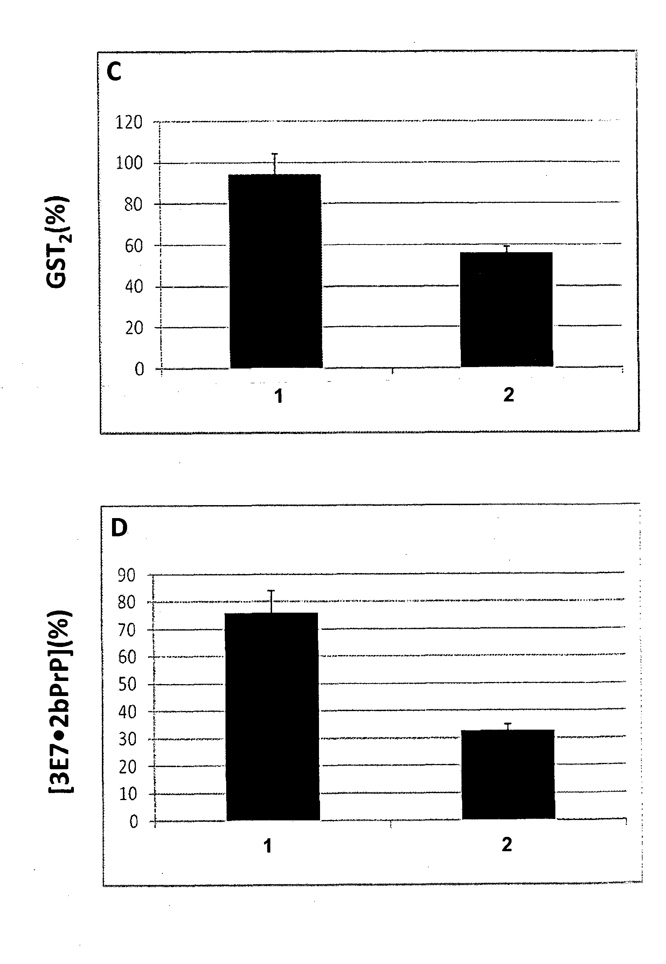

*Figure 5*: *Comparison of cross-linking efficiency.*
   The cross-linking efficiency of 1,1'-(suberoyldioxy) bisazabenzotriazole (SBAT), disuccinimidyl suberate (DSS), dithiobis[succinimidyl propionate] (DSP) and ethylene glycol bis[succinimidylsuccinate] (EGS) (Figure 5A, C), and of Mix 1 (1) and Mix 2 (2) (Figure 5B, D) is compared. Each cross-linking reagent is tested for the interaction analysis of glutathione S-transferase (Figure 5 A,B) and for the immuno-complex [6H4•2bPrP] (Figure 5 C,D). The percentage of GST dimer (GST₂) or [6H4•2bPrP] immuno-complex detected is determined directly from the High-Mass MALDI spectra after completion of the cross-linking reaction after 180 min (Example 9).

### Detailed Description of the Invention

The invention relates to a method of cross-linking proteins and/or other compounds bearing amino functions, wherein the proteins or other compounds or mixtures thereof are incubated with a cross-linker selected from 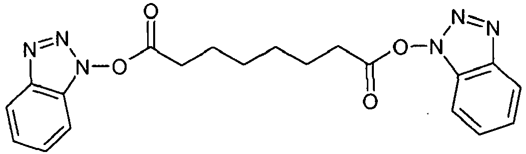 *1*,*1'*-(suberoyldioxy)bisbenzotriazole SBBT (di(1*H*-benzo[*d*][1,2,3]triazol-1-yl) octanedioate), 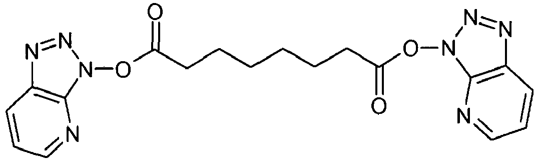 *1,1'*-(suberoyldioxy)bisazabenzotriazole SBAT (di(3H-[1,2,3]triazolo[4,5-b]pyridin-3-yl) octanedioate),
and a mixture of such cross-linkers with each other or with further cross-linkers.

In a particular embodiment, the invention relates to the mentioned method, wherein the cross-linker SBBT or SBAT is isotopically labeled. Examples of isotopic labels considered are ²H (deuterium), ³H (tritium), ¹³C, ¹⁴C, or ¹⁵N. A preferred isotopic label is ²H. Any hydrogen atom of the cross-linker may be replaced by deuterium. Particular examples are cross-linkers, wherein the carbon atoms adjacent to the ester carbonyl functions are deuterated.

Furthermore, the invention relates to the novel compound-*1,1'*-(suberoyldioxy)-bisbenzotriazole (SBBT). Further described are linear C₅- and C₈-C₂₀-alkanedioic acid 7-azabenzotriazol-1-yl diesters (nBAT), 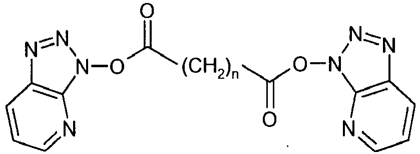 wherein n is 3 and between 6 and 18, in particular *1,1'*-(suberoyldioxy)bisazabenzotriazole (SBAT, n=6), and isotopically labeled SBBT and nBAT.

It is an object of the present invention to provide cross-linking reagents offering better efficiency and kinetic properties for the analysis of supramolecular target-ligand-complexes in either purified multicomponent samples or heterogeneous biological matrices for detection by either mass spectrometry or any other analytical technology (e.g. gel electrophoresis, X-ray crystallography, NMR, or any kind of microscopic or fluorescence based method). In particular the invention relates to a method of analysis of supramolecular target-ligand-complexes, wherein said complexes, for example in purified multicomponent samples or heterogeneous biological matrices, are incubated in aqueous solutions or in organic solvents with SBBT or SBAT or mixtures of such cross-linkers with each other or with further cross-linkers, and the products analyzed in the reaction mixture or after isolation. In particular the invention relates to such method wherein the cross-linked products are analyzed without isolation by High-Mass MALDI mass spectrometry.

In particular, it is an object of the present invention to use a mixture of at least one of the cross-linking reagents SBBT or SBAT together with further cross-linking reagents.

A preferred mixture comprises cross-linkers with 1-hydroxy-7-azabenzotriazole groups as reactive groups of various spacer length, e.g. 3 to 30 A, of the formula 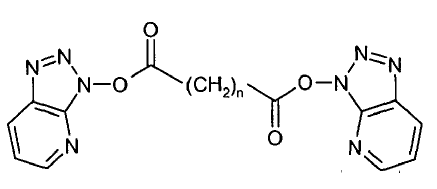 wherein n is 3 and between 6 and 18, preferably between 6 and 14, and in particular 3, 6 and 8. A particular example of a preferred mixture is a mixture of cross-linkers comprising SBAT (8C, n = 6), the corresponding 5C reactive linker *1,1'*-(glutaroyl-dioxy)bisazabenzotriazole (GBAT, di(3H-[1,2,3]triazolo[4,5-b]pyridin-3-yl) glutarate, n = 3) and the corresponding 10C reactive linker *1,1'*-(decanoyldioxy)bisazabenzotriazole (DBAT, di(3H-[1,2,3]triazolo[4,5-b]pyridin-3-yl) decanedioate, n = 8).

Preferred mixtures are composed of SBAT, GBAT and DBAT; SBAT and SBBT; SBAT and DBAT; and SBAT, SBBT and DBAT.

Such mixtures are useful for cross-linking transient complexes and aggregates formed in protein-protein and protein-nucleic acid interactions and subsequent detection of cross-linked protein complexes, including protein aggregates, using High-Mass MALDI mass spectrometry.

In a specific embodiment, the newly developed cross-linking reagents are used for either purified multicomponent samples or heterogeneous biological matrices.

In a further specific embodiment, the supramolecular target-ligand-complex represents a complex of a target molecule with its ligand, wherein said target molecule and/or the ligand can be one particular or a plurality of proteins (e.g. antibody, receptor, enzyme, aggregates), nucleic acids, synthetic organic compounds (e.g. drugs, polymers) carrying amino functions, or particles (e.g. viral particles) carrying such amino functions, and the like. Thus, the complexes formed result from protein-drug, protein-nucleic acid, protein-viral particles, or protein-protein interactions, e.g. antibody-antigen, enzyme-substrate interactions, and the like.

It is a further object of the invention to use the newly developed cross-linking reagents in various biochemistry and molecular/cell biology applications such as biochemical characterization of antibody-antigen interactions (e.g. antibody screening, epitope mapping, reaction kinetics), therapeutic protein development and quality control (protein-PEG analysis; protein aggregate analysis), cellular signaling pathway mapping, as well as in drug discovery applications such as drug screening (e.g. direct target fishing using a bait protein or in competition assays), *in vitro* drug binding assays, drug profiling via mapping protein-protein interactions upon applying stress, as well as complexomics including automated and higher throughput applications.

The cross-linking reagents of the present invention provide increased efficiency and kinetic properties for the direct analysis of intact supramolecular target-ligand complex, allowing more reliable characterization of their nature.

The invention itself will best be understood from the following description of the preferred embodiments of the present invention. It is understood that those skilled in the art may conceive modifications and/or variations to the specific embodiments shown and described herein. The description of preferred embodiments and best mode of the invention known to the applicant at the time of filing the application has been presented and is intended for the purposes of illustration and description. The embodiments demonstrate the principles of the invention and its practical applications and enable others skilled in the art to best utilize the invention in various embodiments and with various modifications as are suited to the particular use contemplated. The scope of the invention is defined by the claims.

The meaning of certain terms and phrases used in the specification and claims are provided below. Unless defined otherwise, all technical and scientific terms used herein have the same meaning as is commonly understood by one of skill in the art to which the subject matter belongs.

The cross-linking reagents of the present invention allow the detection, identification or characterization of intact supramolecular target-ligand-complexes, e.g. from either purified multicomponent sample or heterogeneous biological matrices faster and with higher sensitivity with either mass spectrometry or other analytical technology (e.g. gel or fluorescence based technology, NMR, X ray crystallography and the like).

The present invention is based on the finding that the cross-linking reagents SBBT and SBAT have higher efficiency and react faster than the commercially available cross-linking reagents of the state of the art for the analysis of non-covalently formed, supramolecular target-ligand-complexes, e.g. in either purified multicomponent sample or heterogeneous biological matrices.

The invention relates to SBBT of the formula: 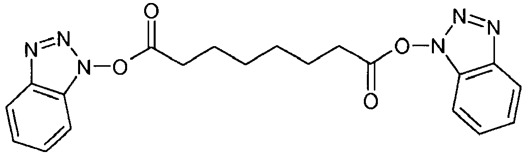 designated 1,1'-(suberoyldioxy) bisbenzotriazole or di(1*H*-benzo[*d*][1,2,3]triazol-1-yl) octanedioate. It may be obtained by reaction of suberic acid or suberic acid derivatives and 1-hydroxybenzotriazole in the presence of reagents activating the carboxylic acid function and known in the art for carboxylic ester formation. In particular the compound may be obtained by reaction of suberic acid dichloride and 1-hydroxybenzotriazole in the presence of an amine base, e.g. triethyl amine.

The invention further relates to isotopically labeled SBBT, wherein the isotopic label is, for example, ²H (deuterium), ³H (tritium), ¹³C, ¹⁴C, or ¹⁵N. A preferred isotopic label is ²H. Any hydrogen atom of SBBT may be replaced by deuterium. A particular example is SBBT, wherein the carbon atoms adjacent to the ester carbonyl functions are deuterated.

Further described are linear C₈-C₂₀-alkanedioic acid 7-azabenzotriazol-1-yl diesters nBAT of the formula 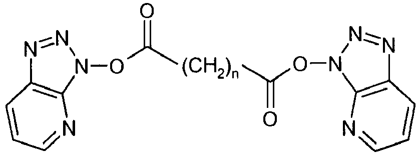 wherein n is 3 or between 6 and 18, preferably between 6 and 14, designated 1,1'-(alkanoyldioxy)bisazabenzotriazole or di(3H-[1,2,3]triazole[4,5-b]pyridine-3-yl) alkanedioate, in particular glutarate (n = 3), suberate (n = 6), azelate (n = 7), sebazate (n = 8), undecanedioate (n = 9), dodecanedioate (n = 10), tridecanedioate (n = 11), tetradecanedioate (n = 12), pentadecanedioate (n = 13), hexadecanedioate (n= 14), heptadecanedioate (n= 15), octadecanedioate (n= 16), nonadecanedioate (n= 17), and icosanedioate (n = 18). These esters may be obtained by reaction of the corresponding dicarboxylic acid or acid derivatives and 1-hydroxy-7-azabenzotriazole in the presence of reagents activating the carboxylic acid function and known in the art for carboxylic ester formation. In particular the compounds may be obtained by reaction of the corresponding dicarboxylic acid dichloride and 1-hydroxy-7-azabenzotriazole in the presence of an amine base, e.g. triethyl amine.

Particularly preferred are *1,1'*-(glutaroyldioxy)bisazabenzotriazole (GBAT, di(3H-[1,2,3]triazolo[4,5-b]pyridin-3-yl) glutarate, n = 3), 1,1'-(suberoyldioxy) bisazabenzotriazole (SBAT, di(3H-[1,2,3]triazolo[4,5-b]pyridin-3-yl) suberate (or octanedioate), n = 6), and *1,1'*-(decanoyldioxy)bisazabenzotriazole (DBAT, di(3H-[1,2,3]triazolo[4,5-b]pyridin-3-yl) decanedioate (or sebazate), n = 8).

The invention further describes isotopically labeled nBAT, wherein the isotopic label is, for example, ²H (deuterium), ³H (tritium), ¹³C, ¹⁴C, or ¹⁵N. A preferred isotopic label is ²H. Any hydrogen atom of nBAT may be replaced by deuterium. A particular example is nBAT, such as the preferred GBAT, SBAT or DBAT, wherein the carbon atoms adjacent to the ester carbonyl functions are deuterated.

As used herein, the term "cross-linker" refers to a small molecule with a molecular weight below 1500 Dalton carrying two or more, preferably two, functional groups able to react efficiently and selectively with amino functions as present in proteins, in particular with amino function presented at the protein surface and with other easily accessible amino functions.

Cross-linkers in the sense of the present invention are described, for example, in S.S. Wong, Chemistry of Protein Conjugation and Cross-Linking (CRC Press 1991). Cross-linkers include both homo- and heteromultifunctional crosslinking agents and include imidoesters, N-hydroxysuccinimide-esters (NHS-esters), maleimides, haloacetyls, pyridyl disulfides, hydrazides, carbodiimides, aryl azides, isocyanates, vinyl sulfones and the like. Common homobifunctional cross-linkers considered are, e.g., alpha-1-acid glycoprotein, 3-[(2-aminoethyl)dithio]propionic acid·HCl, bis-[β-(4-azidosalicyl-amido)ethyl]disulfide, 1,4-bis-maleimidobutane, 1,4-bis-maleimidyl-2,3-dihydroxy-butane, bis-maleimidohexane, bis-maleimidoethane, 1,8-bis-maleimidodiethylene-glycol, 1,11-bis-maleimidotriethyleneglycol, bis[sulfosuccinimidyl] suberate, bis[2-(succinimidooxycarbonyloxy)ethyl]sulfone, 1,5-difluoro-2,4-dinitrobenzene, dimethyl adipimidate·2 HCl, dimethyl pimelimidate·2 HCl, dimethyl suberimidate·2 HCl, 1,4-di-[3'-(2'-pyridyldithio)-propionamido]butane, disuccinimidyl glutarate, dithiobis[succinimidyl-propionate], disuccinimidyl suberate, disuccinimidyl tartarate, dimethyl 3,3'-dithiobis-propionimidate·2 HCl, dithio-bis-maleimidoethane, 3,3'-dithiobis[sulfosuccinimidylpropionate, ethylene glycol bis[succinimidylsuccinate], 1,6-hexane-bis-vinylsulfone, ethylene glycol bis[sulfosuccinimidylsuccinate], p-azidobenzoyl hydrazide, N-(α-maleimidoacetoxy succinimide ester, N-5-azido-2-nitrobenzoyloxysuccinimide, N-[4-(p-azidosalicylamidobutyl]-3'-(2'-pyridyldithio)propionamide, (4-[p-azidosalicylamido]butylamine, N-[ß-maleimidopropionic acid, N-[ß-maleimidopropionic acid]hydrazide·TFA, (N-[β-maleimidopropyloxy]succinimide ester, 1-ethyl-3-[3-dimethylaminopropyl]carbodiimide·HCl, (N-ε-maleimidocaproic acid, [N-ε-maleimidocaproic acid]hydrazide, [N-ε-maleimidocaproyloxy]succinimide ester, N-[κ-maleimidoundecanoic acid, N-[κ-maleimidoundecanoic acid]hydrazide, N-[γ-maleimidobutyryloxy]succinimide ester, succinimidyl 4-[N-maleimidomethyl]cyclohexane-1-carboxy-[6-amidocaproate], succinimidyl 6-(3-[2-pyridyldithio]-propionamido)hexanoate, m-maleimidobenzoyl-N-hydroxysuccinimide ester, 4-(4-N-maleimidophenyl)butyric acid hydrazide·HCl, methyl N-succinimidyl adipate, N-hydroxysuccinimidyl-4-azidosalicylic acid, 3-(2-pyridyl-dithio)propionyl hydrazide, N-[p-maleimidophenyl]isocyanate, N-succinimidyl(4-azidophenyl)-1,3'-dithiopropionate, N-succinimidyl-6-[4'-azido-2'-nitrophenylamino] hexanoate, N-sulfosuccinimidyl-6-[4'-azido-2'-nitrophenylamino] hexanoate, N-succinimidyl-S-acetylthioacetate, N-succinimidyl-S-acetylthiopropionate, succinimidyl 3-[bromoacetamido]propionate, N-succinimidyl iodoacetate, N-succinimidyl[4-iodoacetyl]-aminobenzoate, succinimidyl 4-[N-maleimidomethyl]cyclohexane-1-carboxylate, succinimidyl 4-[p-maleimidophenyl]butyrate, succinimidyl-6-[β-maleimidopropion-amido]hexanoate, 4-succinimidyloxycarbonyl-methyl-α-[2-pyridyldithio]toluene, N-succinimidyl 3-[2-pyridyldithio]-propionamide, [N-ε-maleimidocaproyloxy]sulfosuccinimide ester, N-[γ-maleimidobutyryloxy]sulfosuccinimide ester, N-hydroxysulfosuccinimidyl-4-azidobenzoate, (N-[κ-maleimidoundecanoyloxy]sulfosuccinimide ester, sulfosuccinimidyl 6(3-[2-pyridyldithio]-propionamido)hexanoate, m-maleimidobenzoyl-N-hydroxysulfosuccinimide ester, sulfosuccinimidyl[4-azidosalicylamido]-hexanoate, N-sulfosuccinimidyl(4-azidophenyl)-1,3'-dithiopropionate, sulfosuccinimidyl 2-[7-amino-4-methylcoumarin-3-acetamido]ethyl-1,3'-dithiopropionate, sulfosuccinimidyl 2-[m-azido-o-nitrobenzamido]-ethyl-1,3'-dithiopropionate, sulfosuccinimidyl-2-[p-azidosalicylamido]ethyl-1,3'-dithiopropionate, sulfosuccinimidyl-[perfluoroazidobenzamido]ethyl-1,3'-dithiopropionate, N-sulfosuccinimidyl[4-iodoacetyl]aminobenzoate, succinimidyl 4-[N-maleimidomethyl]cyclohexane-1-carboxylate, sulfosuccinimidyl 4-[p-maleimidophenyl]butyrate, 4-sulfosuccinimidyl-6-methyl-α-(2-pyridyldithio)toluamido]-hexanoate, N-[ε-trifluoroacetylcaproyloxy]succinimide ester, sulfosuccinimidyl-2-[6-biotinamido-2-ρ-azidobenzamidohexanoylamido]ethyl-1,3'-dithiopropionate, (β-[tris-(hydroxymethyl)phosphino]propionic acid, (tris[2-maleimidoethyl]amine), and tris-succinimidyl aminotriacetate. Further cross-linkers considered are formaldehyde, glutaraldehyde, or glyoxal.

As used herein, the term "higher efficiency" refers to the property of the newly developed cross-linkers to increase the amount of cross-linked supramolecular target-ligand-complex after the cross-linking reaction.

As used herein "faster reaction" refers to the property of the newly developed cross-linkers to increase the rate of cross-linking supramolecular target-ligand-complexes for short incubation time, e.g from 1 to 30 minutes.

As used herein the term "spacer length" refers to the length of the carbon chain (in A) separating the two reactive groups of a bifunctional cross-linking molecule.

As used herein, the term "supramolecular target-ligand-complexes" refers to complexes arising from the specific binding of a target molecule with its binding ligand, wherein said target molecule and/or binding ligand can be one particular or a plurality of proteins (e.g. antibody, receptor, enzyme or protein aggregates), nucleic acids, synthetic organic compounds (e.g. drugs, polymers) or particles (e.g. viral particles) and the like, to form said supramolecular target-ligand-complexes, such as protein-protein, protein-nucleic acid, protein-drug, protein-viral particles, antibody-antigen, substrate-enzyme complexes and the like.

As used herein, the term "target" or "target molecule" refers to a higher mass molecule, which typically may be found in a biological source but may also be a synthetic molecule that is based on or derived from a naturally occurring molecule, and in particular includes proteins (both antibody and non-antibody proteins), polypeptides, glycopolypeptides, phosphopolypeptides, peptidoglycans, polysaccharides, peptidomimetics, lipids, carbohydrates, polynucleotides and other naturally occurring or synthetic macromolecules, preferably proteins, polypeptides, polysaccharides, peptidomimetics, lipids, carbohydrates, and polynucleotides, more preferably proteins, polypeptides, peptidomimetics, and polynucleotides. A target can be derived from a natural source or chemically synthesized.

As used herein, the term "ligand" (or "binding ligand") refers to a molecule that can specifically bind to its target, such as an antigen to an antibody, a substrate to an enzyme, a polypeptide to an epitope, or a protein to a protein or to a group of proteins. The molecules described as ligand and target can be used interchangeably. Thus a ligand can be essentially any type of molecule such as a small molecule drug, peptide or polypeptide, protein, nucleic acid or oligonucleotide, carbohydrate such as oligosaccharides, viral particles, or any other small organic derived compound and synthetic macromolecule. A ligand can be derived from a natural source or chemically synthesized.

As used herein, the term "purified multicomponent sample" refers to any sample containing a heterogeneous or homogeneous mixture of proteins, polypeptides, glycopolypeptides, phosphopolypeptides, peptidoglycans, polysaccharides, peptidomimetics, lipids, carbohydrates, polynucleotides or organic compounds, which has been purified in part or completely.

As used herein, the term "heterogeneous biological matrices" refers to any crude reaction mixture including mixtures obtained from dissolution of a solid material such as a tissue, cells, or a cell pellet; biological fluid such as urine, blood, saliva, amniotic fluid, or exudates from a region of infection or inflammation; a cell extract, or biopsy sample; or mixtures obtained from a living source, for example, an animal such as a human or other mammal, a plant, a bacterium, a fungus or a virus.

The above defined terms "purified multicomponent sample" and "heterogeneous biological matrices" may also refer to synthetically obtained mixtures whereby a series of potential binding ligands is contacted with the target molecule.

As used herein, the term "analyze" means to identify or detect the presence, absenceor change of, or determine the identity of such covalently stabilized supramolecular target-ligand-complexes as intact ions.

The cross-linking reagents of the present invention are in particular very useful as they allow analysis of weak protein interactions requiring faster and more efficient cross-linking reactions.

Furthermore, the cross-linkers disclosed herein provide an important tool in various analytical applications which are of great importance in drug development and diagnostics. These analytical applications include characterization of the antibody-antigen interactions with special regard to the specificity, cross reactivity, binding strength, kinetics and stoichiometry of the interaction, a topic with significance in reagent development and antibody drug optimization; epitope mapping, with significance in basic molecular biology research and reagent development; enzyme induced polymerization or cleavage of proteins as well as post-translational modification, e.g phosphorylation, dephosphorylation, glycolysation, acetylation, methylation, ubiquitination, etc., of interacting proteins with significance in signaling pathway mapping; lead finding assay, e.g. ligand fishing using a bait protein or competition assay with significance in drug discovery; drug profiling assay, e.g. mapping protein complex formation in the cell upon application of the drug or other stress factors, with significance in biomarker discovery, validation and early drug profiling; higher throughput, direct analysis of protein-protein interactions-complexomics, interactomics or systems biology; any automated or higher throughput analysis to determine properties such as the binding kinetics, binding sites, identification or the stoichiometry of the components, either *in vitro* or *in vivo;* comparison of multiple cell lines, some of which may have supramolecular complexes which have been expressed, excited or perturbed, to differenciate presence of identified or unknown complex(es); any detection of phosphorylation / dephosphorylation sites, such as protein kinases, using these methods and measurement of protein transport throughout another entity, such as through a cell, or part of a cell.

### Examples

### Synthesis of cross-linkers

### Example 1: 1,1'-(Suberoyldioxy) bisbenzotriazole SBBT (di(1H-benzo[d][1,2,3]triazol-1-yl) octanedioate)

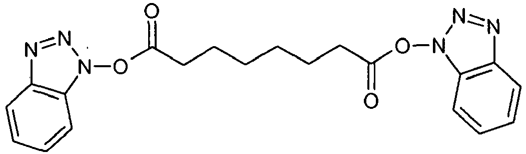

The coupling between suberic chloride and 1-hydroxybenzotriazole was done in analogy to the method described for adipoyl chloride (Ueda et al., Journal of Polymer Science, Part a, Polymer Chemistry 1974, 14(11):2665-2673). A solution of 1-hydroxy-benzotriazole (1.0 g; 7 mmol) and triethylamine (1.2 mL; 9 mmol) in dry THF (5 mL) is dropwise mixed with suberic chloride (0.6 mL; 4 mmol) at 0°C. The solution is then stirred for 30 min and poured into 200 mL of H₂O. The precipitate is filtered and dried.

### Example 2: 1,1'-(Suberoyldioxy)bisazabenzotriazole SBAT (di(3H-[1,2,3]triazolo[4,5-b]pyridin-3-yl) octanedioate)

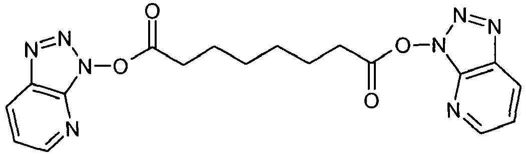

A solution of 3*H*-[1,2,3]triazolo[4,5-b]pyridin-3-ol (1.08 g; 8 mmol) and of triethylamine (1.2 mL; 9 mmol) in dry THF (5 mL) is treated with suberic chloride (0.6 mL; 4 mmol) and stirred for 30 min at 0°C. The solution is poured into H₂O and the precipitate filtered.

Compounds of Examples 1 and 2 are analyzed by ¹H NMR, on a Varian Mercury 300 or a Varian Gemini instrument. For mass spectrometric characterization, spectra are acquired on a quadrupole time-of-flight mass spectrometer (Q-ToF ULTIMA, Waters, Manchester, UK) equipped with an automated chip-based nanoESI system (Nanomate 100, Advion Biosciences, Ithaca, NY, USA).

### Example 3: Sample preparation

The protein complexes to be stabilized are cross-linked with an excess of the cross-linking mixture in a total volume of 10 µL. Each cross-linker is freshly solubilized in dimethyl formamide (DMF) at 2 mg/mL, and is added to an aliquot of the protein or peptide solution at a 50 fold molar excess. The sample and the cross-linker are incubated between 2 and 180 min at room temperature. After cross-linking, 1 µL of the sample containing the protein complexes is directly mixed with 1 µL of matrix solution. The matrix solution, freshly made, contains sinapic acid (10 mg/mL) in acetonitrile: water (1:1, v/v) acidified with 0.1 % (v/v) trifluoroacetic acid (TFA). After mixing, 1 µL of the sample is deposited on the sample plate for MALDI-MS analysis.
The specificity of the binding between the protein complex and the cross-linker is tested by adding ubiquitin (5 µL, 30 µM) to a glutathione S-transferase (GST) solution (5 µL, 15 µM) and incubating the mixture with one of the synthetic cross-linkers for 120 min at room temperature.
For the kinetic studies, the cross-linking reaction is analyzed at 25°C, at different incubation times, by rapidly mixing an aliquot of the sample with the matrix and spotting onto the MALDI plate. The crystallization with matrix is assumed to stop the binding reaction.

### Example 4: Mass spectrometry

MALDI mass spectra are acquired on a time-of-flight mass spectrometer (Reflex IV, Bruker Daltonics GmbH, Bremen, Germany) equipped with a High-Mass detection system (HM1, CovaIX AG, Zurich, Switzerland). The high-mass detection system provides high sensitivity with low saturation in the high mass range up to >1 MDa. Typical accelerating voltages are 20 kV, HV2, the gain voltage on the detector is set at 2.8 kV, and the delayed extraction time is automatically adapted by the instrument's software according to the selected mass range. Spectra are averaged over 300 laser shots at different locations of the sample. For the kinetics study, spectra are first background subtracted and smoothed with the software ComplexTracker 1.0 (CovaIX AG, Zürich, Switzerland).

### Example 5: High-Mass MALDI Mass spectral analysis of a non-covalent (GST dimer) protein-protein interaction cross-linked with disuccinimidyl suberate (DSS) or 1,1'-(suberoyldioxy) bisazabenzotriazole SBAT) after 2 min incubation time.

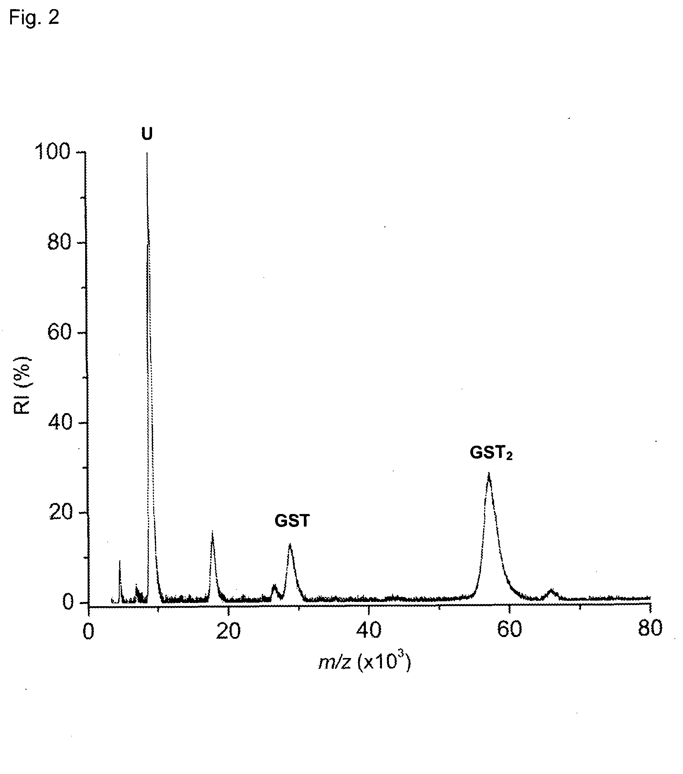

This example demonstrates the High-Mass MALDI analysis of glutathione S-transferase (GST) dimer under non-crosslinked conditions (Figure 2A) and after 2 min incubation time of the protein with the cross-linkers DSS (Figure 2B) or SBAT (Figure 2C). The cross-linking reactions involve 10 µL of 1 µM of GST reacting with 1 µL of a solution of DSS (50 µM in DMF) or SBAT (50 µM in DMF). Analysis is performed using a MALDI mass spectrometer (Reflex IV, Bruker Daltonics GmbH, Bremen, Germany) equipped with a High-Mass detection system (HM1, CovaIX AG, Zurich, Switzerland). Samples are prepared using 1 µL of cross-linked GST mixed with 1 µL of sinapic acid (10 mg/mL in 70% acetonitrile:30% water: 0.1% trifluoroacetic acid) and spotting 1 µL using dried droplet technique.

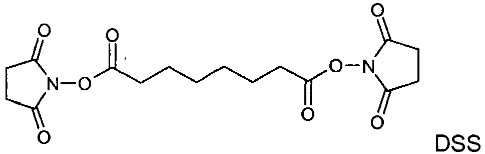

### Example 6: Specificity Analysis: High-Mass MALDI Mass spectral analysis of a mixture of glutathione S-transferase (GST) and ubiquitin cross-linked with 1,1'-(suberoyldioxy) bisazabenzotriazole (SBAT) after 120 min incubation time.

This example demonstrates the faster kinetic properties of cross-linker SBAT when compared to commercially available cross-linking reagents disuccinimidyl suberate (DSS, bis(2,5-dioxopyrrolidin-1-yl) octanedioate), dithiobis succinimidyl propionate (DSP, bis(2,5-dioxopyrrolidin-1-yl) 3,3'-disulfanediyldipropanoate) and ethylene glycol bis succinimidyl succinate (EGS, bis(2,5-dioxopyrrolidin-1-yl)-ethane-1,2-diyl disuccinate). High-Mass MALDI analysis of a mixture of glutathione S-transferase (GST) and ubiquitin is performed after 120 min cross-link reaction with SBAT (Figure 3). The cross-linking reactions involve 10 µL of 1 µM of GST mixed with 2 µM of ubiquitin and reacting with 1 µL of a solution of SBAT (50 µM in DMF). Analysis is performed using a MALDI mass spectrometer (Reflex IV, Bruker Daltonics GmbH, Bremen, Germany) equipped with a High-Mass detection system (HM1, CovaIX AG, Zurich, Switzerland). Samples are prepared using 1 µL of cross-linked product mixed with 1 µL of sinapic acid (10 mg/mL in 70% acetonitrile: 30% water: 0.1% trifluoroacetic acid) and spotting 1 µL using dried droplet technique.

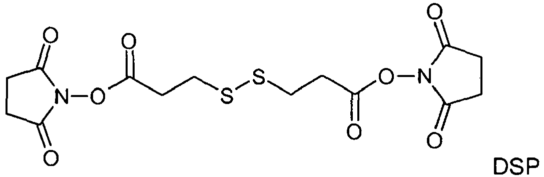

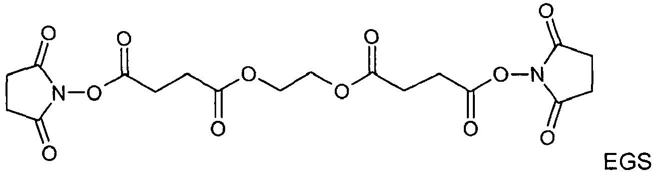

### Example 7: Comparative study of the kinetics properties between 1,1'-(suberoyldioxy) bisazabenzotriazole (SBAT), disuccinimidyl suberate (DSS), dithiobis[succinimidyl propionate] (DSP), and ethylene glycol bis[succinimidylsuccinate] (EGS).

This example demonstrates the faster kinetic properties of cross-linker SBAT when compared to commercially available cross-linking reagents disuccinimidyl suberate (DSS, bis(2,5-dioxopyrrolidin-1-yl) octanedioate), dithiobis[succinimidyl propionate] (DSP, bis(2,5-dioxopyrrolidin-1-yl) 3,3'-disulfanediyldipropanoate) and ethylene glycol [bissuccinimidylsuccinate] (EGS, bis(2,5-dioxopyrrolidin-1-yl)-ethane-1,2-diyl disuccinate). High-Mass MALDI mass spectrometric analysis of glutathione S-transferase (GST) is performed after different incubation times between 0 and 10 min (Figure 4A) and 0 and 240 min (Figure 4B) with different cross-linkers DSS, DSP, EGS, and SBAT. After each incubation, the sample is analyzed by High-Mass MALDI mass spectrometry and the % of GST dimer is directly determined by integration of the peak on the mass spectrum. The cross-linking reactions involve 50 µL of GST (1 µM) reacting with 5 µL of a solution of DSS (50 µM in DMF), DSP (50 µM in DMF), EGS (50 µM in DMF) or SBAT (50 µM in DMF). After each incubation, 1 µL of the cross-linked sample is mixed with 1 µL of sinapic acid (10 mg/mL in 70% acetonitrile:30% water: 0.1% trifluoroaceticaAcid). 1 µL is spotted using dried droplet technique. Analysis is performed using a MALDI mass spectrometer (Reflex IV, Bruker Daltonics GmbH, Bremen, Germany) equipped with a High-Mass detection system (HM1, CovaIX AG, Zurich, Switzerland).

### Example 8: Comparative study of the kinetic properties for multi-length cross-linking mixture having 1-hydroxy-7-azabenzotriazole groups as reactive groups and multi-length cross-linking mixture of commercially available cross-linkers.

This example demonstrates the faster kinetic properties of a mixture containing 1,1'-(suberoyldioxy) bisazabenzotriazole (SBAT, di(3H-[1,2,3]triazolo[4,5-b]pyridin-3-yl) octanedioate), glutaroyldioxy bisazabenzotriazole (GBAT, di(3H-[1,2,3]triazolo[4,5-b]-pyridin-3-yl) glutarate) and decanoyldioxy bisazabenzotriazole (DBAT, di(3H-[1,2,3]triazolo[4,5-b]pyridin-3-yl) decanedioate (Mix 1) compared with a mixture containing disuccinimidyl suberate (DSS, bis(2,5-dioxopyrrolidin-1-yl) octanedioate), dithiobis[succinimidyl propionate] (DSP, bis(2,5-dioxopyrrolidin-1-yl) 3,3'-disulfane-diyldipropanoate) and ethylene glycol bis[succinimidylsuccinate] (EGS, bis(2,5-dioxopyrrolidin-1-yl)'-ethane-1,2-diyl disuccinate) (Mix 2). For this experiment, three different cross-linkers having 1-hydroxy-7-azabenzotriazole groups as reactive groups are synthesized in order to obtain 3 different spacer lengths separating the reactive groups (GBAT: 8.8 A; SBAT: 13.2 A; DBAT: 16.1 A), and mixed in equimolar ratio. A mixture of 3 different cross-linkers commercially available (DSS, DSP and EGS) having different spacer length (from 11.4 to 16.1 A) is also prepared in equimolar ratio. High-Mass MALDI mass spectrometric analysis of glutathione S-transferase (GST) or [3E7•2bPrP] immuno-complex is performed after different incubation time (0 to 10 min, Figure 5A and C, and 0 to 180 min, Figure 5B and D) with Mix 1 or Mix 2. After each incubation time, the integration of the peak corresponding to GST dimer or [3E7•2bPrP] is determined to evaluate the efficiency of the cross-linking reaction. The cross-linking reactions involve 50 µL of GST (1 µM) or 50 µL of a protein solution containing 2 µM of monoclonal antibody 6H4 and 4 µM of antigen bPrP reacting with 5 µL of a solution containing Mix1 (50 µM in DMF) or Mix 2 (50 µM in DMF). After each incubation, 1 µL of the cross-linked sample is mixed with 1 µL of sinapic acid (10 mg/mL in 70% acetonitrile: 30% water: 0.1% trifluoroacetic acid). 1 µL is spotted using dried droplet technique. Analysis is performed using a MALDI mass spectrometer (Reflex IV, Bruker Daltonics GmbH, Bremen, Germany) equipped with a High-Mass detection system (HM1, CovaIX AG, Zurich, Switzerland).

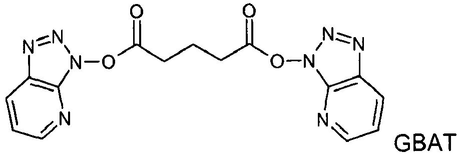

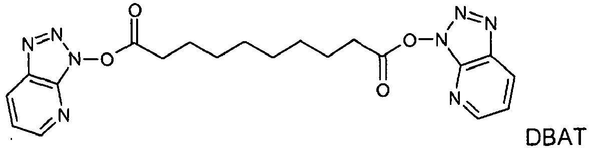

### Example 9: Efficiency of the cross-linking reaction

This example demonstrates the higher efficiency of the newly developed cross-linking reagents when compared to commercially available cross-linkers. For evaluating the efficiency of the newly developed cross-linkers, two solutions containing protein complexes (GST and 1 E7-bPrP) are tested. 10 µL of GST (1 µM) or 10 µL of a protein solution containing 2 µM of monoclonal antibody 1 E7 and 4 µM of antigen bPrP are mixed with 1 µL of a solution containing DSS (50 µM in DMF), DSP (50 µM in DMF), EGS (50 µM in DMF), SBAT (50 µM in DMF), Mix 1 (50 µM in DMF) or Mix 2 (50 µM in DMF). The cross-linkers are incubated 180 min with the protein samples to ensure complete cross-linking reaction. After incubation, 1 µL of the cross-linked sample is mixed with 1 µL of sinapic acid (10 mg/mL in 70% acetonitrile: 30% water: 0.1% trifluoroacetic acid). 1 µL is spotted using dried droplet technique. Analysis is performed using a MALDI mass spectrometer (Reflex IV, Bruker Daltonics GmbH, Bremen, Germany) equipped with a High-Mass detection system (HM1, CovaIX AG, Zurich, Switzerland). For each protein solution and each cross-linking reagent, the peak corresponding to the cross-linked protein complex on the mass spectrum is integrated to evaluate the cross-linking efficiency.

## Claims

1. A method of cross-linking proteins and/or other compounds bearing amino functions, wherein the proteins or other compounds or mixtures thereof are incubated with a cross-linker selected from 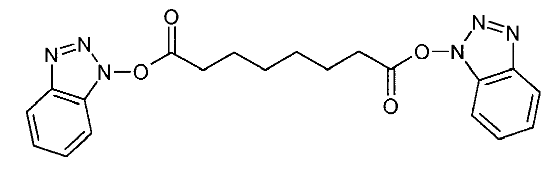 *1,1'*-(suberoyldioxy)bisbenzotriazole (SBBT), 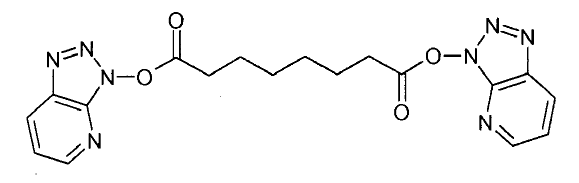 *1,1'*-(suberoyldioxy)bisazabenzotriazole (SBAT),
and a mixture of such cross-linkers with each other or with further cross-linkers.

2. A method of analysis of supramolecular target-ligand-complexes either in purified multicomponent samples or heterogeneous biological matrices, wherein the samples or matrices are incubated in aqueous solutions or in organic solvents with a cross-linker selected from 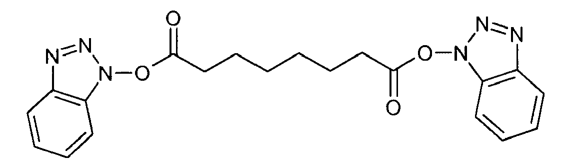 *1,1'*-(suberoyldioxy)bisbenzotriazole (SBBT), 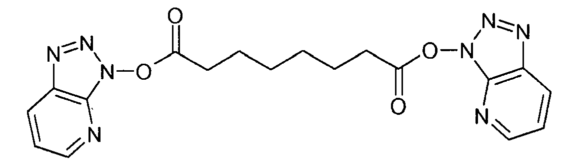 *1,1'*-(suberoyldioxy)bisazabenzotriazole (SBAT), and a mixture of such cross-linkers with each other or with further cross-linkers, and the products analyzed in the reaction mixture or after isolation.

3. The method of claim 1 or 2 wherein the selected cross-linker is isotopically labeled.

4. The method of claim 2 or 3 wherein the cross-linked products are analyzed by mass spectrometry, gel electrophoresis, fluorescence based technologies, NMR, X-ray crystallography or microscopy.

5. The method of claim 2 or 3 wherein the cross-linked products are analyzed without isolation by High-Mass MALDI mass spectrometry.

6. The method of anyone of claims 1 to 5 wherein a mixture of cross-linkers SBBT or SBAT with each other or with further cross-linkers is used.

7. The method of claim 6 wherein the mixture comprises one or more cross-linkers with a 1-hydroxy-7-azabenzotriazole group.

8. The method of claim 6 wherein the mixture comprises *1,1-*(suberoyldioxy)bisazabenzotriazole (SBAT), *1,1'*-(glutaroyldioxy)bisazabenzotriazole (GBAT), and *1,1'-*(decanoyldioxy)bisazabenzotriazole (DBAT).

9. The method of claim 6 wherein the mixture comprises *1,1'*-(suberoyldioxy)bisazabenzotriazole (SBAT) and *1,1'*-(decanoyldioxy)bisazabenzotriazole (DBAT).

10. The method of claim 6 wherein the mixture comprises *1,1'*-(suberoyldioxy)bisazabenzotriazole (SBAT), *1,1'*-(suberoyldioxy)bisbenzotriazole (SBBT), and *1,1'-*(decanoyldioxy)bisazabenzotriazole (DBAT).

11. *1,1'*-(Suberoyldioxy)bisbenzotriazole (SBBT) of formula

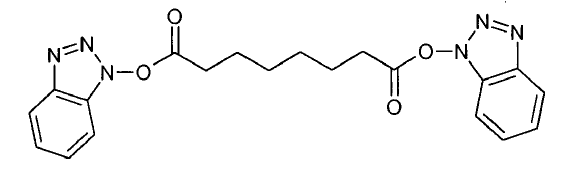

## Patentansprüche

1. Verfahren zur Vernetzung von Proteinen und/oder anderer Verbindungen, die Aminofunktionen besitzen, worin die Proteine oder anderen Verbindungen oder Mischungen davon mit einem Vernetzungsmittel ausgewählt aus 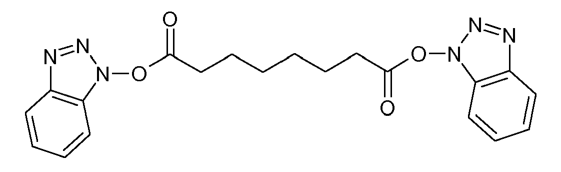 *1,1'*-(Suberoyldioxy)bisbenzotriazol (SBBT), 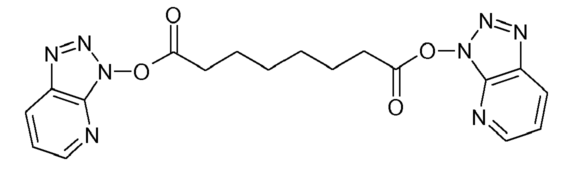 *1,1'*-(Suberoyldioxy)bisazabenzotriazol (SBAT),
und einer Mischung solcher Vernetzungsmittel untereinander oder mit anderen Vernetzungsmitteln inkubiert wird.

2. Verfahren zur Analyse von supramolekularen Zielobjekt-Ligand-Komplexen entweder in gereinigten Vielkomponenten-Proben oder in heterogenen biologischen Grundmassen, worin die Proben oder Grundmassen in wässrigen Lösungen oder in organischen Lösungsmitteln mit einem Vernetzungsmittel ausgewählt aus 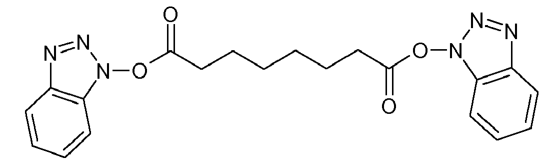 *1,1'*-(Suberoyldioxy)bisbenzotriazol (SBBT), 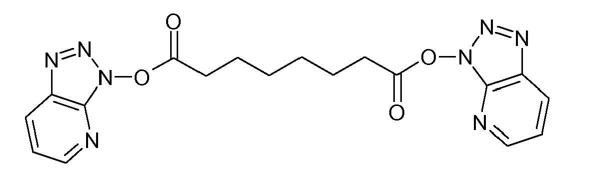 *1,1'*-(Suberoyldioxy)bisazabenzotriazol (SBAT), und einer Mischung solcher Vernetzungsmittel untereinander oder mit anderen Vernetzungsmitteln inkubiert wird, und die Produkte in der Reaktionsmischung oder nach Isolierung analysiert werden.

3. Verfahren gemäss Anspruch 1 oder 2, worin die ausgewählten Vernetzungsmittel isotopisch markiert sind.

4. Verfahren gemäss Anspruch 2 oder 3, worin die vernetzten Produkte mit Massenspektrometrie, Gel-Elektrophorese, auf Fluoreszenz basierenden Technologien, Kernspinresonanz (NMR), Röntgenkristallographie oder Mikroskopie analysiert werden.

5. Verfahren gemäss Anspruch 2 oder 3, worin die vernetzten Produkte ohne Isolierung mit MALDI Massenspektrometrie für hohe Massen analysiert werden.

6. Verfahren gemäss irgendeinem der Ansprüche 1 bis 5, worin eine Mischung der Vernetzungsmittel SBBT oder SBAT untereinander oder mit anderen Vernetzungsmitteln verwendet wird.

7. Verfahren gemäss Anspruch 6, worin die Mischung ein oder mehrere Vernetzungsmittel mit einer 1-Hydroxy-7-azabenzotriazol-Gruppe umfasst.

8. Verfahren gemäss Anspruch 6, worin die Mischung *1,1'*-(Suberoyldioxy)bis-azabenzotriazol (SBAT), *1,1'*-(Glutaroyldioxy)bisazabenzotriazol (GBAT) und *1,1'-*(Decanoyldioxy)bisazabenzotriazol (DBAT) umfasst.

9. Verfahren gemäss Anspruch 6, worin die Mischung *1,1'*-(Suberoyldioxy)bis-azabenzotriazol (SBAT) und *1,1'*-(Decanoyldioxy)bisazabenzotriazol (DBAT) umfasst.

10. Verfahren gemäss Anspruch 6, worin die Mischung *1,1'*-(Suberoyldioxy)bis-azabenzotriazol (SBAT), *1,1'*-(Suberoyldioxy)bisbenzotriazol (SBBT) und *1,1'-*(Decanoyldioxy)bisazabenzotriazol (DBAT) umfasst.

11. *1,1'*-(Suberoyldioxy)bisbenzotriazol (SBBT) der Formel

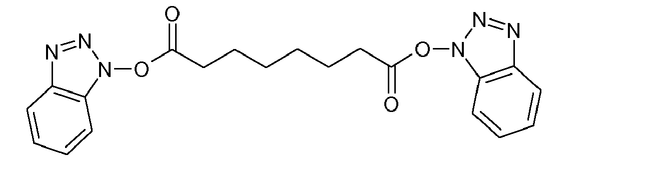

## Revendications

1. Procédé pour la réticulation de protéines et/ou autres composés possédant des fonctions amino, dans lequel les protéines ou autres composés ou leurs mélanges sont incubés avec un agent de réticulation sélectionné parmi: 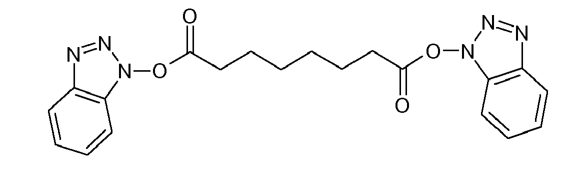 1,1'-(suberoyldioxy)bisbenzotriazole (SBBT), 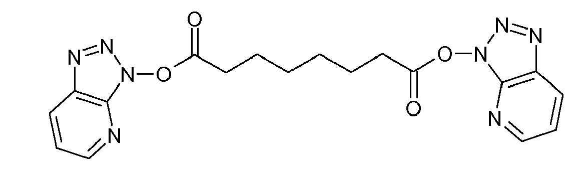 1,1'-(suberoyldioxy)bisazabenzotriazole (SBAT),
et un mélange de tels agents de réticulation les uns avec les autres ou avec d'autres agents de réticulation.

2. Procédé d'analyse de complexes ligands cibles supramoléculaires soit dans des échantillons multi-composants purifiés ou matrices biologiques hétérogènes, dans lequel les échantillons ou matrices sont incubés dans des solutions aqueuses ou dans des solvants organiques avec un agent de réticulation sélectionné parmi: 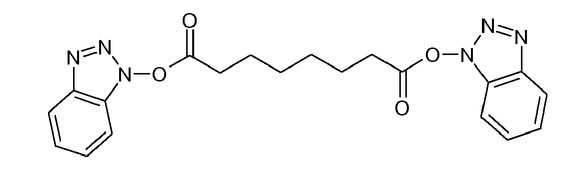 1,1'-(suberoyldioxy)bisbenzotriazole (SBBT), 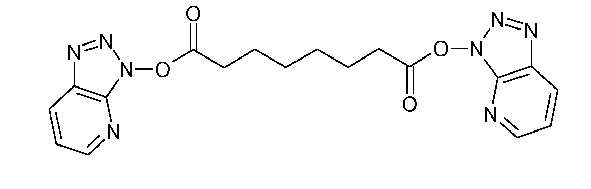 *1,1'*-(suberoyldioxy)bisazabenzotriazole (SBAT),
et un mélange de tels agents de réticulation les uns avec les autres ou avec d'autres agents de réticulation, et les produits étant analysés dans un mélange réactionnel ou après isolation.

3. Le procédé de la revendication 1 ou 2, dans lequel l'agent de réticulation sélectionné est isotopiquement marqué.

4. Le procédé de la revendication 2 ou 3, dans lequel les produits réticulés sont analysés par spectrométrie de masse, électrophorèse sur gel, technologies basées sur la fluorescence, résonance magnétique nucléaire (RMN), radiocristallographie ou microscopie.

5. Le procédé de la revendication 2 ou 3, dans lequel les produits réticulés sont analysés sans isolation par spectrométrie de masse MALDI à masse élevée.

6. Le procédé de l'une quelconque des revendications 1 à 5, dans lequel un mélange d'agents de réticulation SBBT ou SBAT les uns avec les autres ou avec d'autres agents de réticulation est utilisé.

7. Le procédé de la revendication 6, dans lequel le mélange comprend un ou plusieurs agents de réticulation avec un groupe 1-hydroxy-7-azabenzotriazole.

8. Le procédé de la revendication 6, dans lequel le mélange comprend *1,1'*-(suberoyldioxy)bis-azabenzotriazole (SBAT), *1,1'*-(glutaroyldioxy)bisazabenzotriazole (GBAT), et *1,1-*(decanoyldioxy)bisazabenzotriazole (DBAT).

9. Le procédé de la revendication 6, dans lequel le mélange comprend *1,1'*-(suberoyldioxy)bis-azabenzotriazole (SBAT) et *1,1'*-(decanoyldioxy)bisazabenzotriazole (DBAT).

10. Le procédé de la revendication 6, dans lequel le mélange comprend *1,1'*-(suberoyldioxy)-bisazabenzotriazole (SBAT), *1,1'*-(suberoyldioxy)bisbenzotriazole (SBBT), et *1,1'-*(decanoyldioxy)bisazabenzotriazole (DBAT).

11. *1,1'*-(Suberoyldioxy)bisbenzotriazole (SBBT) ayant la formule
